(19) **Europäisches Patentamt / European Patent Office / Office européen des brevets**

(11) **EP 1 450 287 B1**

(12) **EUROPEAN PATENT SPECIFICATION**

(45) Date of publication and mention of the grant of the patent:
**16.11.2016 Bulletin 2016/46**

(51) Int Cl.:
*A61B 5/00* *(2006.01)*   *G06T 5/50* *(2006.01)*
*G06T 7/00* *(2006.01)*

(21) Application number: **03258211.6**

(22) Date of filing: **23.12.2003**

(54) **Method of extracting region of interest from tongue image and health monitoring method and apparatus using the tongue image**

Verfahren zur Extrahierung eines interessanten Gebiets aus einem Zungenbild und Verfahren und Gerät zur Überwachung des Gesundheitzustandes unter Verwendung des Zungenbildes

Méthode d'extraction d'une région d'intérêt dans une image de la langue et méthode et appareil de surveillance de l'état de santé utilisant cette image de la langue

(84) Designated Contracting States:
**DE FR GB**

(30) Priority: **28.12.2002 KR 2002085915**

(43) Date of publication of application:
**25.08.2004 Bulletin 2004/35**

(73) Proprietor: **Samsung Electronics Co., Ltd.**
**Suwon-si, Gyeonggi-do, 443-742 (KR)**

(72) Inventors:
- **Kim, Tae-woo**
  **Songpa-gu**
  **Seoul (KR)**
- **Yoon, Gil-won**
  **Seongdong-gu**
  **Seoul (KR)**
- **Lee, Jeong-whan**
  **Suwon-si**
  **Gyeonggi-do (KR)**
- **Shin, Sang-hoon**
  **Seongnam-si**
  **Bundang-gu**
  **Gyeonggi-do (KR)**

(74) Representative: **O'Callaghan, Robert James et al**
**Elkington and Fife LLP**
**Prospect House**
**8 Pembroke Road**
**Sevenoaks, Kent TN13 1XR (GB)**

(56) References cited:
**US-A- 5 872 859**

- **PATENT ABSTRACTS OF JAPAN vol. 2002, no. 03, 3 April 2002 (2002-04-03) -& JP 2001 314376 A (RES DEV CORP OF JAPAN; OGITA BIO SCIENCE KENKYUSHO:KK; NIPPON ELECTRON), 13 November 2001 (2001-11-13)**
- **LEI JIAN LIU; JIAN FENG LU; JING YU YANG; KE LIU; YONG GE WU; SHI JING LI: "Efficient segmentation of nuclei in different color spaces" PROCEEDINGS OF THE SPIE - THE INTERNATIONAL SOCIETY FOR OPTICAL ENGINEERING, 26 July 1995 (1995-07-26), pages 773-778, XP002360888 USA**
- **PATENT ABSTRACTS OF JAPAN vol. 1995, no. 04, 31 May 1995 (1995-05-31) -& JP 07 000398 A (ZENICHI OGITA; others: 01), 6 January 1995 (1995-01-06)**

**Description**

The present invention relates to health monitoring, and more particularly, to a method of extracting a region of interest from a tongue image and a health monitoring method and apparatus using characteristic factors extracted from the region of interest in the tongue image.

With overall improvements in life environment and conditions, personal interest in health has constantly increased. As a result, a lot of home medical equipments for allowing people to easily monitor their personal health have been researched and developed.

Many factors can be used to diagnose the condition of a human body. Among them, blood pressure or blood glucose is usually used due to easy measurement. In addition to these factors, it has been recently acknowledged that a state of tongue can also provide a lot of information about internal organs. For example, when people are healthy, they have pink tongues with a thin, white tongue coat. When a liver or a digestive organ do not work properly, a tongue is swollen, becomes thin, shrinks due to dryness, is yellow or gray, has a thick tongue coat, or is slick without any tongue coat.

Recently, there have been published many papers on the relation between a tongue state and a visceral cancer or a chill-fever syndrome. Some of them are Chuang-Cine Chiu, "A Novel Approach Based on Computerized image Analysis for Traditional Chinese Medical Diagnosis of the Tongue," Computer Methods and Programs in Biomedicine 61, pp. 77-89, 2000, Yang Cai, "A novel Imaging System for Tongue Inspection," IEEE Instrumentation and Measurement Technology Conference, pp. 159-163, 2002, Yao and Paotia, "Comparison of TCM Tongue Images with Gastroscopy Images," Shangdong S&T Publisher, 1996 (in Chinese), and Tadashi, Watsuji, et al., "Medical Application of Fuzzy Theory to the Diagnostic System of Tongue Inspection in Traditional Chinese Medicine," IEEE International Fuzzy Systems Conference Proceedings, pp. 145-148, 1999.

However, equipment for detecting the incipient stage of the invasion of a disease and the progress of the disease based on a state of the tongue, such as a tongue coat, color, or appearance, is very expensive and can be mostly found in hospitals. As such, the conventional equipment is not suitable for people to personally monitor their health.

JP 2001-314376 discloses a system whereby a diagnosis can be made on the basis of tongue images stored in a database.

Li and Yuen (C.H. Li, P.C. Yuen, "Tongue image matching using color content", Pattern Recognition 35 (2002) 407-419) disclose a method of tongue image matching using color content. The tongue region is extracted manually from the images.

According to an aspect of the present invention, there is provided a method of extracting a region of interest from a tongue image of a person whose health state is to be determined, the method comprising: constructing a database in which template tongue images for a plurality of persons are stored, including a template tongue image for the person whose health state is to be determined, wherein each template tongue image corresponds to personal information and is indicated with regions of interest; separating a tongue area from a tongue image acquired from the person whose health state is to be determined; matching the separated tongue area with a template tongue image stored in the database; and extracting the region of interest from the separated tongue area using the matched template tongue image.

According to another aspect of the present invention, there is provided a health monitoring apparatus using a tongue image, comprising: a tongue image database in which a tongue image obtained from each person, a template tongue image set for the person, and a result of determining a health state of the person with respect to at least one characteristic factor are linked to one another; a tongue image acquisition unit which acquires a tongue image from a person, whose health state is to be determined; a region-of-interest extractor which separates a tongue area from the tongue image provided from the tongue image acquisition unit and extracts a region of interest from the tongue area using template matching between the tongue area and a template tongue image for the person stored in the tongue image database; a characteristic extractor which generates data regarding at least one characteristic factor with respect to the region of interest extracted by the region-of-interest extractor; a comparator which compares the data regarding the characteristic factor generated with respect to the region of interest by the characteristic extractor with a characteristic factor stored in the tongue image database with respect to the region of interest; and a health state determiner which determines the person's health state based on the result of comparison provided from the comparator and informs the person of the result of determination.

The present invention thus provides a method of extracting a region of interest, such as the tip, middle part, edge, or root of the tongue, from a tongue image.

The present invention also provides a health monitoring method and apparatus using at least one characteristic factor of a region of interest extracted from a tongue image.

The above and other features and advantages of the present invention will become more apparent by describing in detail a preferred embodiment thereof with reference to the attached drawings in which:

FIG. 1 is a block diagram of a health monitoring apparatus using a tongue image, according to an embodiment of the present invention;

FIG. 2 is a detailed block diagram of a tongue image processing unit shown in FIG. 1;

FIG. 3 is a flowchart of an operation of a region-of-interest extractor shown in FIG. 2;

FIGS. 4A through 4D illustrate a generation and matching of a template image stored in a tongue image database;

FIG. 5 illustrates the tip, middle part, left edge, right edge, and root of the tongue;

FIGS. 6A and 6B show examples of a tongue image acquired via the tongue image acquisition unit shown in FIG. 1 and a basic tongue image stored in the tongue image database shown in FIG. 1;

FIGS. 7A and 7B show examples of a tongue tip extracted as a region of interest from the tongue images shown in FIGS. 6A and 6B;

FIGS. 8A and 8B show examples of a tongue middle extracted as a region of interest from the tongue images shown in FIGS. 6A and 6B;

FIGS. 9A and 9B are graphs showing histograms in a Hue Saturation Intensity (HSI) color coordinate system with respect to the tongue tips shown in FIGS. 7A and 7B; and

FIGS. 10A and 10B are graphs showing histograms in an HSI color coordinate system with respect to the tongue middles shown in FIGS. 8A and 8B.

Hereinafter, a preferred embodiment of the present invention will be described in detail with reference to the attached drawings.

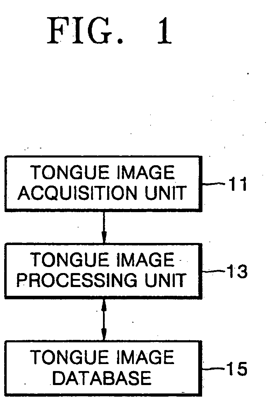

FIG. 1 is a block diagram of a health monitoring apparatus using a tongue image, according to an embodiment of the present invention. The health monitoring apparatus includes a tongue image acquisition unit 11, a tongue image processing unit 13, and a tongue image database 15.

The tongue image acquisition unit 11 is implemented as a typical digital camera, such as an exclusive camera for photographing the tongue, a camera attached to a personal computer, or a camera attached to a mobile communication terminal. The whole tongue of a person to be diagnosed is photographed every day or before he/she eats food when necessary using the tongue image acquisition unit 11. The tongue image acquisition unit 11 acquires a tongue image and transmits it to the tongue image processing unit 13. It is preferable that the tongue is photographed under constant illumination in order to reduce color distortion of a tongue image. Also, it is preferable that the color of the tongue image is compensated using a color checker. It is assumed that the person sticks out the tongue in almost the same form, for example, to have almost the same length or make the same angle, every time the tongue is photographed.

The tongue image processing unit 13 extracts a region of interest, such as a tongue tip, a tongue middle, a tongue edge, or a tongue root, from a tongue image provided from the tongue image acquisition unit 11 using thresholding and template matching with a basic tongue image stored in the tongue image database 15, and compares the region of interest extracted from the currently acquired tongue image with that in the basic tongue image based on predetermined characteristic factors. In addition, the tongue image processing unit 13 can compare a tongue proper or coat in the region of interest with the basic tongue image based on predetermined characteristic factors and determine a health state based on Chinese medicine.

The tongue image database 15 includes personal information including a unique identifier of each person, a tongue image acquired from each person through the tongue image acquisition unit 11, a template image in which regions of interest are set, and characteristic factor data with respect to each region of interest in each health state or on each date, which are associated with one another.

In the health monitoring apparatus, the tongue image acquisition unit 11, the tongue image processing unit 13, and the tongue image database 15 can be integrated into a single apparatus or can be separately implemented. When they are integrated into a single apparatus, the health monitoring apparatus can be implemented in a personal mobile communication equipment such as a cellular phone or a personal digital assistant (PDA). In this case, the tongue image database 15 stores personal information, tongue information, and tongue characteristics, with respect to a tongue image of the owner of a mobile communication equipment. When they are separately implemented, data transmission and reception is performed among them using wired or wireless communication, and the tongue image processing unit 13 and the tongue image database 15 can be implemented in a health case service center or a personal computer. The health care service center may be a server of a mobile communication equipment company or a server of a hospital. In the meantime, only the tongue image database 15 can be separately implemented, and when necessary, a tongue image can be wirely or wirelessly transmitted from the tongue image database 15 to a remote oriental medical doctor, and the oriental medical doctor can diagnose a health state and give advice based on Chinese medicine.

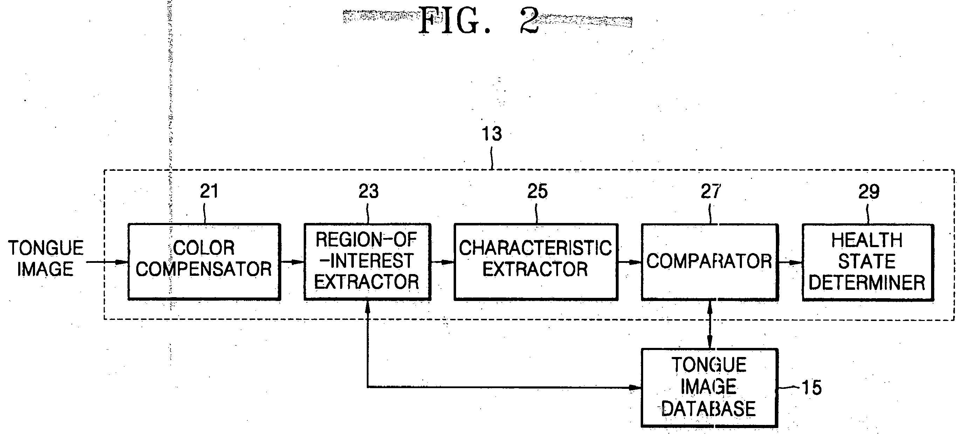

FIG. 2 is a detailed block diagram of the tongue image processing unit 13 shown in FIG. 1. The tongue image processing unit 13 includes a color compensator 21, a region-of-interest extractor 23, a characteristic extractor 25, a comparator 27, and a health state determiner 29.

The color compensator 21 compensates a tongue image for a color distortion due to ambient illumination during acquisition of the tongue image via the tongue image acquisition unit 11. For color compensation, the tongue image acquisition unit 11 generates and stores a color checker including a plurality of colors to be displayed on the tongue image when acquiring the tongue image, and the color compensator 21 compares the color checker included in the tongue image with the color checker provided from the tongue image acquisition unit 11, obtains a color difference value between the two color checkers, and determines a degree of compensation based on the color difference value. Here, the color compensator 21 may be omitted. In other words, the color compensator 21 is optional according to an environment in which the tongue is photographed.

The region-of-interest extractor 23 matches the tongue image received from the color compensator 21 with a basic template image of a relevant person, which is stored in the tongue image database 15, and extracts a region of interest such as a tongue tip, a tongue middle, a tongue left edge, a tongue right edge, or a tongue root.

The characteristic extractor 25 detects characteristic factors of the region of interest extracted by the region-of-interest extractor 23. The characteristic extractor 25 can detect characteristic factors such as a histogram of color values in a Hue Saturation Intensity (HSI) color coordinate system, an average of the color values, a deviation, entropy, and texture.

The comparator 27 compares the characteristic factors of the region of interest of the tongue image, which are detected by the characteristic extractor 25, with characteristic factors of the region of interest of the basic template image, which are stored in the tongue image database 15 according to health states or dates.

The health state determiner 29 has a data range for each characteristic factor with respect to a health state, the range being set in advance, and determines the health state of a current person using the result of the comparison provided from the comparator 27. For example, H, S, and I values obtained from an HSI histogram of a tongue tip, which are stored with respect to a healthy state and a fatigue state in the tongue image database 15, can be compared with H, S, and I values obtained from an HSI histogram of a tongue tip extracted from a currently acquired tongue image, and a current health state can be determined based on the result of the comparison. In addition, states of tongue proper and coat in the region of interest can be detected based on the result of comparison of characteristic factors so as to determine a current health state based on Chinese medicine.

Table 1 shows internal organs corresponding to each part of the tongue and health states based on Chinese medicine.

Table 1

| Items | Description |
| --- | --- |
| Organs | ► Tongue tip: Upper heater, Upper part of the stomach - Heart and lungs<br>► Tongue middle: Middle heater, Middle part of the stomach - Spleen and Stomach<br>► Tongue edge: None - Liver and gallbladder<br>► Tongue root: Lower heater, Lower part of the stomach - Kidney |
| Health criteria | Health state is determined based on states of tongue proper and coat.<br>► Tongue proper: Mainly determines deficiency or excess of organs.<br>► Tongue coat: Mainly determines the clarity or turbidity of the stomach "Gi" and properties of pathogen.<br>(Healthy state: Tongue has reddish and glossy proper and a thin white coat and is not too dry nor too damp.) |
| Tongue proper | ► White: White tongue proper indicates deficiency and cold syndrome or anemia.<br>► Reddish: Reddish tongue tip indicates abundant heat in the upper heater or flaring-up of the heart fire. Reddish tongue edge indicates a hepatic heat syndrome. Scarlet tongue proper indicates steaming fever due to warm-heart, i.e., flaming-up of fire due to deficiency of Eum. Crimson appears mostly when pathogenic heat enters the nutrient "Gi".<br>► Purple: The three heaters have extreme heat. Dark blue indicates accumulation of stagnated blood. Damp tongue having light purple with blue indicates that cold pathogen enters the liver and kidneys.<br>► Dark blue: Slippery tongue indicates deficiency of Eum. Dryness indicates the syndrome of blood stagnation. These two characteristics indicate that disease has been advanced. |
| Tongue coat | ► White coat: White, sticky, and slippery coat indicates that body has damp-phlegm inside. White, sticky, and thick coat indicates abundant damp turbidity. State as if tongue was shaken powder off indicates that turbidity of epidemic disease is high. White coat is mostly exterior syndrome in exogenous diseases. |

(continued)

| Items | Description |
|---|---|
|  | ► Yellow coat: Light yellow and non-dry coat indicates that pathogen starts to enter the body. Yellow and sticky coat indicates damp-heat. Yellow and stained like coat indicates that damp is more vigorous than heat. Dark yellow and cracked coat indicates that heat is more vigorous than damp. |
|  | ► Dark gray coat: Gray and lightly moistened and slippery coat with light viscosity and stagnation indicates Eum-cold due to accumulation of fluids or direct attack of exogenous pathogenic factor. Dry and dark coat indicates that abundant heat damages a thin body fluid and extreme fire dries up water. Moistened and slippery coat indicates deficiency of Yang and excessive Eum, that is, dominance of water over fire. |

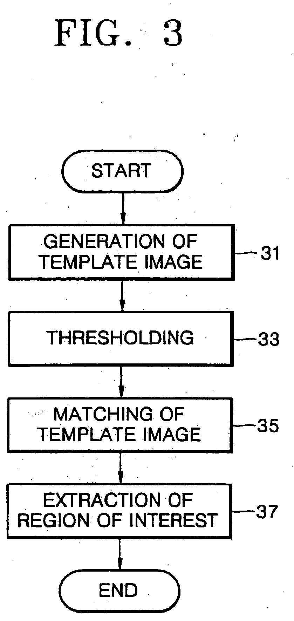

FIG. 3 is a flowchart of an operation of the region-of-interest extractor 23 shown in FIG. 2. The operation includes generation of a template image (31), segmentation of an inner mouth area (33), matching of the template image (35), and extraction of a region of interest (37).

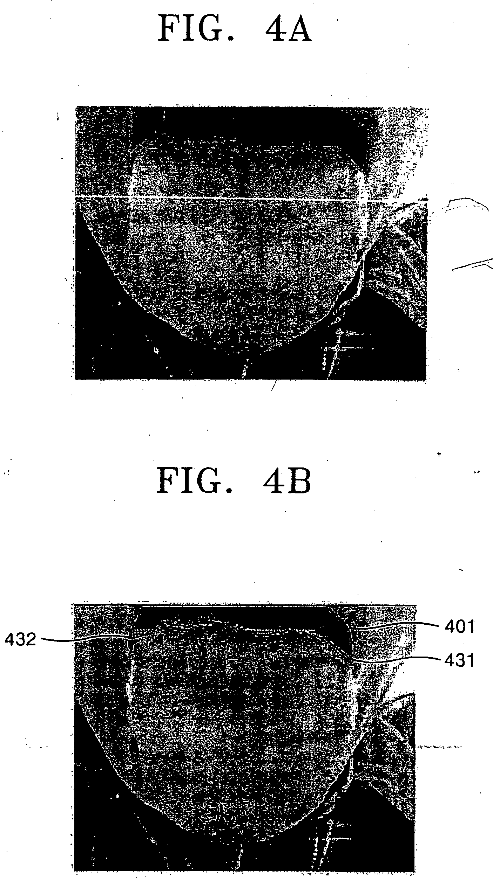

In step 31, a template image of the tongue of a person is generated and stored in the tongue image database 15. Step 31 will be described in detail with reference to FIGS. 4A through 4D.

In order to generate a template image of the tongue, thresholding is performed on a tongue image acquired via the tongue image acquisition unit 11 so as to segment the tongue image into an inner mouth area, which roughly occupies 1/4 from the top of the tongue image, and a tongue area. Thresholding of the tongue image shown in FIG. 4A is performed according to Formula (1).

$$g(x) = 1, \; f(x) \leq T$$
$$g(x) = 0, \; f(x) > T \qquad \qquad ...(1)$$

Here, $f(x)$ is a pixel value at a pixel position x in the tongue image and has a range of $0 \leq f(x) \leq 255$, $g(x)$ is a pixel value indicating the inner mouth area and may be 0 or 1, and $T$ is a threshold value which may be obtained experimentally and, form example, may be set to 70. In the meantime, at least one among Red/Green/Blue (RGB) color values of each pixel can be used as $f(x)$. Alternatively, for example, $I(x)$ corresponding to brightness in an HSI color coordinate system can be used instead of $f(x)$. In this case, in order to obtain $I(x)$ trom RGB coior values, RGB/HSI color coordinate system conversion formula shown in Formula (2) is used. The RGB and HSI color coordinate systems are used in this embodiment, but other various color coordinates systems such as CMY, YIQ, and HSV color coordinate systems can be used.

$$I = F \frac{R + G + B}{3}$$

$$H = \frac{F}{2\pi} \cos^{-1} \left\{ \frac{\frac{1}{2}[(R - G) + (R - B)]}{[(R - G)^2 + (R - B)(G - B)]^{1/2}} \right\} \qquad ...(2)$$

$$S = F \left\{ 1 - \frac{3}{R + G + B} \min(R, G, B) \right\}$$

Here, $0 \leq R, G, B \leq 1$, and F is a constant, typically set to 255.

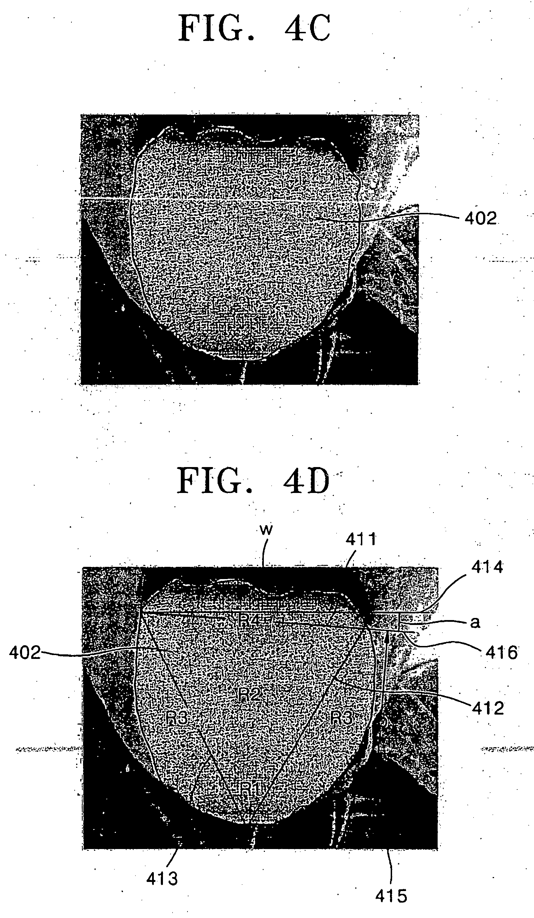

As the result of performing thresholding using Formula (1) on the tongue image shown in FIG. 4A provided from the tongue image acquisition unit 11, the tongue image is segmented into an inner mouth area 401 where $g(x)$ for each pixel is 1, as shown in FIG. 4B, and a tongue area 402 where $g(x)$ for each pixel is 0, as shown in FIG. 4C.

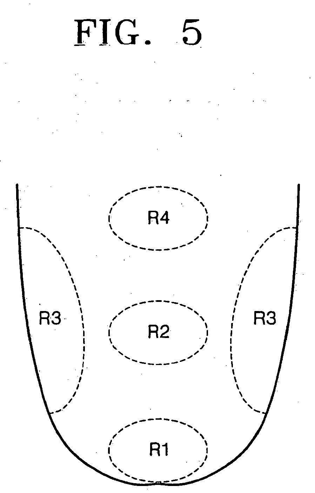

Next, as shown in FIG. 4D, a triangle composed of three sides 411, 412, and 413, which are defined by two opposite end points 431 and 432 of the inner mouth area 401 and a lower end point of the tongue area 402 segmented from the tongue image shown in FIG. 4A, is set. Regions of interest are set using the triangle, as shown in FIG. 5. In FIG. 5, a reference character R1 denotes a tongue tip, a reference character R2 denotes a tongue middle, a reference character R3 denotes a tongue edge, and a reference character R4 denotes a tongue root. In FIG. 4D, a tongue middle R2 is set to a predetermined region at the center of the tongue area 402. A tongue root R4 is set to a predetermined region at the center of the side 411. A left tongue edge R3 and a right tongue edge R3 are respectively set to predetermined outer regions at the centers of the respective sides 413 and 412. A tongue tip R1 is set to a predetermined region near the point where the two sides 413 and 412 meet.

In step 33, a tongue image is acquired from a person whose health state is to be determined using the tongue image acquisition unit 11, and thresholding is applied to the acquired tongue image in the same manner as in the generation of the template image in step 31 so as to segment the acquired tongue image into the inner mouth area 401 and the tongue area 402.

In step 35, a tongue width "w" in the tongue area 402 is obtained based on the opposite end points 431 and 432 of the inner mouth area 401, a rotation angle "a" of the tongue is obtained based on a horizontal line 414 starting from the left end of the side 411 and a line 416 extending from the side 411, and a tongue length "d" is obtained as the distance between parallel lines 415 and 416. The person's tongue image is matched with the template image using template characteristic factors, such as the tongue width "w", the rotation angle "a", and the tongue length "d".

In step 37, regions of interest, including at least one of the tongue tip R1, the tongue middle R2, the left tongue edge R3, the right tongue edge R3, and the tongue root R4, are extracted from the acquired tongue image using the matched template image.

Hereinafter, a procedure for determining a health state will be described with an example of a basic tongue image stored in the tongue image database 15 and an example of a tongue image currently acquired by the tongue image acquisition unit 11.

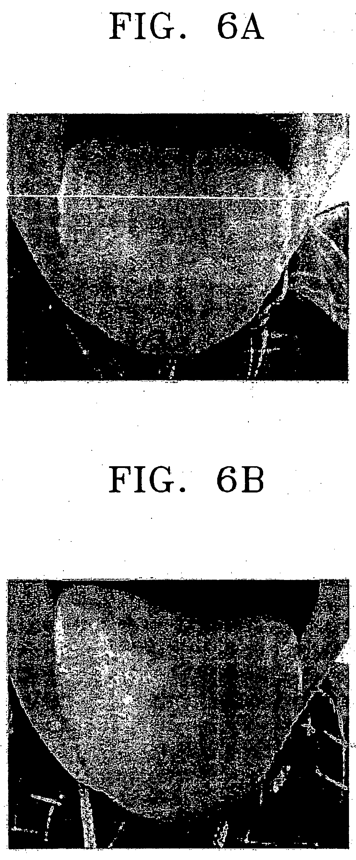

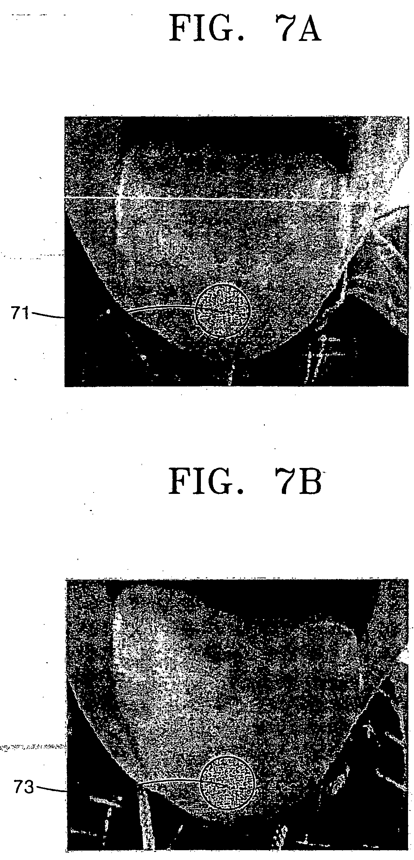

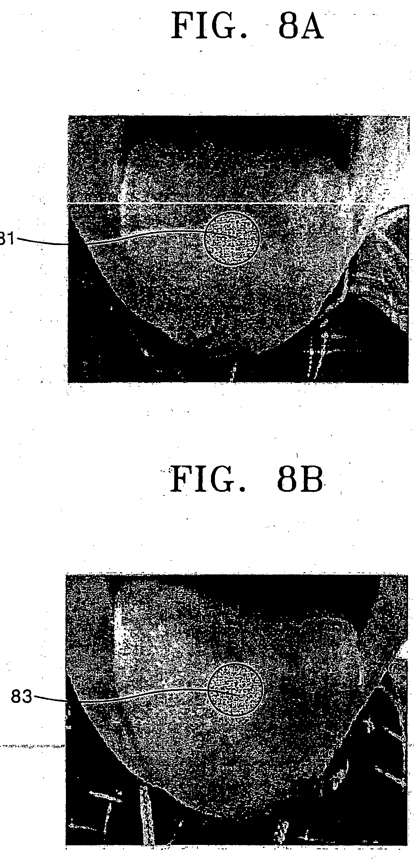

FIG. 6A is a basic tongue image stored in the tongue image database 15,corresponding to a low fatigue state. FIG. 6B is a tongue image acquired from a person in high fatigue state using the tongue image acquisition unit 11. FIGS. 7A and 7B show examples of a tongue tip extracted as a region of interest 71 from the tongue image shown in FIG. 6A and a tongue tip extracted as a region of interest 73 from the tongue image shown in FIG. 6B. FIGS. 8A and 8B show examples of regions of interest 81 and 83 corresponding to a tongue middle, which are respectively extracted from the tongue images respectively shown in FIGS. 6A and 6B.

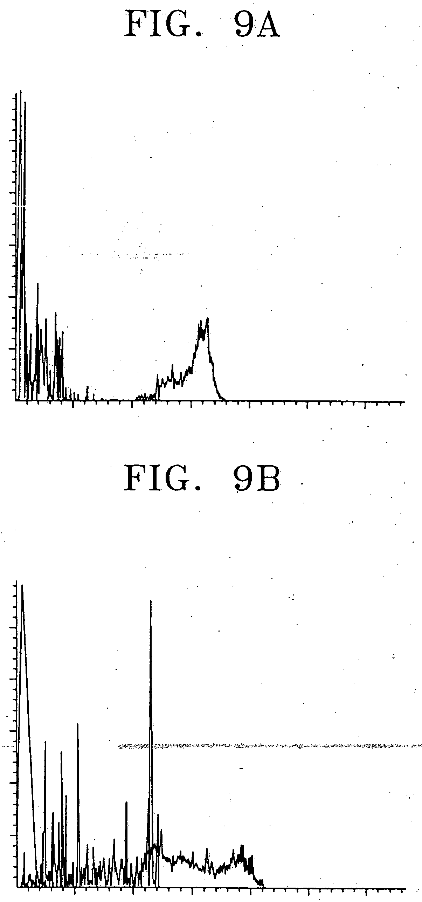

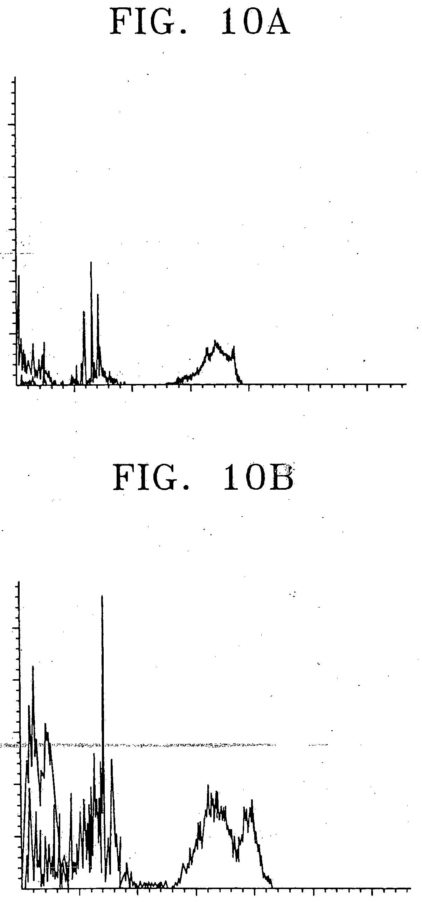

FIG. 9A shows a histogram of color values in an HSI color coordinate system with respect to the region of interest 71 corresponding to the tongue tip shown in FIG. 7A. FIG. 9B shows a histograms of color values in an HSI color coordinate system with respect to the region of interest 73 corresponding to the tongue tip shown in FIG. 7B. FIG. 10A shows a histogram of color values in an HSI color coordinate system with respect to the region of interest 81 corresponding to the tongue middle shown in FIG. 8A. FIG. 10B shows a histograms of coior vaiues in an HSI color coordinate system with respect to the region of interest 83 corresponding to the tongue middle shown in FIG. 8B.

Table 2 shows examples of characteristics of the regions of interest in the tongue images, i.e., average ± standard deviation with respect to each of H, S, any I, obtained from the histograms shown in FIGS. 9A through 10B.

Table 2

| Division | Tonque tip | Tongue middle |
|---|---|---|
| A little fatigue state (FIG. 6A) | H = 20.23 ± 19.71<br>S = 7.51 ± 4.08<br>I = 156.64 ± 15.97 | H = 68.59 ± 14.42<br>S = 12.19 ± 9.41<br>I = 176.78 ± 12.96 |
| Very fatigue state (FIG. 6B) | H = 73.26 ± 36.75<br>S = 8.38 ± 4.36<br>I = 157.55 ± 31.14 | H = 53.08 ± 25.63<br>S = 15.16 ± 8.75<br>I = 177.07 ± 18.99 |

Referring to Table 2, the H, S, and I values are different according to health states, and particularly, the H value greatly changes depending on a health state.

The present invention can be realized as a code which is recorded on a computer readable recording medium and can be read by a computer. For example, a method of extracting a region of interest from a tongue image can be implemented by recording on a computer readable recording medium a first program for constructing a database in which template images are stored, each template image corresponding to personal information of a person; a second program for performing thresholding using a predetermined threshold value on a tongue image, which is acquired from a person whose health state is to be determined using a predetermined image pickup device, so as to segment the tongue image into an inner mouth area and a tongue area; a third program for matching the acquired tongue image with the template image using a tongue width, a rotation angle of the tongue, and a tongue length which are calculated from the tongue area; and a fourth program for extracting a region of interest, such as a tongue middle, a tongue root, a tongue edge, or a tongue tip, from the tongue area using the matched tongue image.

In the meantime, the computer readable recording medium may be any type of recording medium on which data which can be read by a computer system can be recorded, for example, a ROM, a RAM, a CD-ROM, a magnetic tape, a floppy disc, or an optical data storage device. The present invention can also be realized as carrier waves (for example, transmitted through Internet). Alternatively, computer readable recording media are distributed among computer systems connected through a network so that the present invention can be realized as a code which is stored in the recording media and can be read and executed in the computers. Functional programs, codes, and code segments for implementing the present invention can be easily inferred by programmers in the art of the present invention.

As described above, according to the present invention, a health monitoring apparatus using a tongue image is implemented in a mobile communication equipment with a digital camera so that a person's health state can be easily diagnosed based on a change in a state of a region of interest, such as a tongue middle, a tongue root, a tongue edge, or a tongue tip, extracted from a tongue image acquired from the person using the digital camera. Accordingly, the present invention can contribute to personal health care.

In addition, the person's tongue image acquired using the camera can be wirely or wirelessly transmitted to a remote health care service center, in which a tongue image processing unit and a tongue image database are installed, so that a health state can be determined through comparison of characteristic factors or can be more accurately diagnosed by an expert such as an oriental medical doctor.

Moreover, the tongue image processing unit and the tongue image database can be implemented in a server of a mobile communication equipment company or a hospital, and therefore, the company or the hospital can be effectively promoted, and a value added can be created by providing additional services.

Although a few embodiments of the present invention have been shown and described, it will be appreciated by those skilled in the art that changes may be made in these elements without departing from the principles of the invention, the scope of which is defined in the appended claims and their equivalents.

**Claims**

1.  A method of extracting a region of interest from a tongue image of a person whose health state is to be determined, the method comprising:

    constructing a database (15) in which template tongue images for a plurality of persons are stored, including a template tongue image for the person whose health state is to be determined, wherein each template tongue image corresponds to personal information and is indicated with regions of interest;
    separating a tongue area (402) from a tongue image acquired from the person whose health state is to be determined;
    matching the separated tongue area (402) with a template tongue image stored in the database (15); and
    extracting the region of interest from the separated tongue area (402) using the matched template tongue image.

2.  The method of claim 1, further comprising compensating for color distortion of the tongue image using a color checker before separating the tongue area (402).

3.  The method of claim 1 or 2, wherein separating the tongue area (402) comprises performing thresholding on each pixel in the tongue image using a predetermined threshold value so as to define an inner mouth area and a tongue area (402) and separating the tongue area (402) from the tongue image.

4.  The method of claim 3, wherein the thresholding is performed using at least one among RGB color values of each pixel in the tongue image.

5.  The method of claim 3, wherein the thresholding is performed using an intensity value obtained by converting RGB color values of each pixel in the tongue image into HSI color values.

6.  The method of any one of claims 1 to 5, wherein matching the separated tongue area (402) comprises:

    calculating a tongue width (w), a rotation angle of a tongue (a), and a tongue length (b) with respect to the separated tongue area (402); and
    matching the separated tongue area (402) with the template tongue image using the tongue width (w), the rotation angle (a), and the tongue length (b).

7.  The method of any one of claims 1 to 6, wherein the region of interest includes at least one of a tongue middle, a tongue root, a tongue edge, and a tongue tip.

8. A health monitoring method for a person whose health state is to be determined, the method comprising the method of extracting a region of interest from a tongue image according to any one of claims 1 to 7, wherein the database (15) further includes a tongue image obtained from the person and a result of determining a health state of the person with respect to at least one characteristic factor, and wherein the tongue image, the template tongue image for the person and the result of determining are linked to one another, the method further comprising:

detecting at least one characteristic factor from the extracted region of interest; and
determining the health state of the person based on a change in a tongue state, which is detected from a comparison between the detected characteristic factor and a characteristic factor searched from the database with respect to the region of interest in different health states.

9. The health monitoring method of claim 8, wherein the characteristic factor is an average of H, S, or I values obtained from a histogram of an HSI color coordinate system for the region of interest.

10. A computer readable recording medium on which is recorded a program for performing the method of any one of the preceding claims.

11. The computer readable recording medium of claim 10, wherein each step of the method is performed by a separate program.

12. A health monitoring apparatus using a tongue image, comprising:

a tongue image database (15) in which a tongue image obtained from each person, a template tongue image in which regions of interest are set for the person, and a result of determining a health state of the person with respect to at least one characteristic factor are linked to one another;
a tongue image acquisition unit (11) which acquires a tongue image from a person, whose health state is to be determined;
a region-of-interest extractor (23) which separates a tongue area (402) from the tongue image provided from the tongue image acquisition unit (11) and extracts a region of interest from the tongue area using template matching between the tongue area and a template tongue image for the person stored in the tongue image database (15);
a characteristic extractor (25) which generates data regarding at least one characteristic factor with respect to the region of interest extracted by the region-of-interest extractor (23);
a comparator (27) which compares the data regarding the characteristic factor generated with respect to the region of interest by the characteristic extractor (25) with a characteristic factor stored in the tongue image database (15) with respect to the region of interest; and
a health state determiner (29) which determines the person's health state based on the result of comparison provided from the comparator (27) and informs the person of the result of determination.

13. The health monitoring apparatus of claim 12, further comprising a color compensator (21) which compensates for color distortion of the tongue image acquired by the tongue image acquisition unit (11) using a color checker and provides the compensated tongue image to the region-of-interest extractor (23).

14. The health monitoring apparatus of claim 12 or 13, wherein the region-of-interest extractor (23) performs template matching using a tongue width, a rotation angle of a tongue, and a tongue length which are calculated from the tongue area (402).

15. The health monitoring apparatus of any one of claims 12 to 14, wherein the health monitoring apparatus is integrated into a mobile communication equipment with a digital camera.

16. The health monitoring apparatus of any one of claims 12 to 15, wherein the health monitoring apparatus is integrated into a personal computer with a digital camera.

17. The health monitoring apparatus of any one of claims 12 to 16, wherein all of the components except for the tongue image acquisition unit (11) are implemented in a remote health care service center, and the tongue image acquired by the tongue image acquisition unit (11) is transmitted to the health care service center by wired or wireless communication.

**18.** The health monitoring apparatus of claim 17, wherein the health care service center is a server of a mobile communication equipment company or a hospital.

**Patentansprüche**

**1.** Verfahren zum Ausziehen einer Region von Interesse von einem Zungenbild einer Person, deren Gesundheitszustand bestimmt werden soll, wobei das Verfahren Folgendes umfasst:

Aufbauen eine Datenbank (15), in der Vorlagen-Zungenbilder für eine Vielzahl von Personen gespeichert sind, einschließlich eines Vorlagen-Zungenbilds für die Person, deren Gesundheitszustand bestimmt werden soll, wobei jedes Vorlagen-Zungenbild persönlichen Angaben entspricht und mit Regionen von Interesse gekennzeichnet ist;
Heraustrennen eines Zungenbereichs (402) aus einem Zungenbild, das von der Person, deren Gesundheitszustand bestimmt werden soll, erfasst wurde;
Abgleichen des herausgetrennten Zungenbereichs (402) mit einem in der Datenbank (15) gespeicherten Vorlagen-Zungenbild; und
Ausziehen der Region von Interesse aus dem herausgetrennten Zungenbereich (402) unter Verwendung des abgeglichenen Vorlagen-Zungenbilds.

**2.** Verfahren nach Anspruch 1, weiter umfassend das Korrigieren von Farbverfälschung des Zungenbilds unter Verwendung eines Farbprüfers vor dem Heraustrennen des Zungenbereichs (402).

**3.** Verfahren nach Anspruch 1 oder 2, wobei das Heraustrennen des Zungenbereichs (402) das Ausführen von Schwellenwertbildung an jedem Pixel in dem Zungenbild unter Verwendung eines vorausbestimmten Schwellenwerts, um einen inneren Mundbereich und einen Zungenbereich (402) zu definieren und das Heraustrennen des Zungenbereichs (402) aus dem Zungenbild umfasst.

**4.** Verfahren nach Anspruch 3, wobei die Schwellenwertbildung unter Verwendung von mindestens einem aus RGB-Farbwerten jedes Pixels in dem Zungenbild ausgeführt wird.

**5.** Verfahren nach Anspruch 3, wobei die Schwellenwertbildung unter Verwendung eines Intensitätswerts ausgeführt wird, der durch Umwandeln von RGB-Farbwerten jedes Pixels in dem Zungenbild in HSI-Farbwerte erhalten wird.

**6.** Verfahren nach einem der Ansprüche 1 bis 5, wobei das Abgleichen des herausgetrennten Zungenbereichs (402) Folgendes umfasst:

Berechnen einer Zungenbreite (w), eines Drehwinkels einer Zunge (a), und einer Zungenlänge (b) in Bezug auf den herausgetrennten Zungenbereich (402); und
Abgleichen des herausgetrennten Zungenbereichs (402) mit dem Vorlagen-Zungenbild unter Verwendung der Zungenbreite (w), des Drehwinkels (a) und der Zungenlänge (b).

**7.** Verfahren nach einem der Ansprüche 1 bis 6, wobei die Region von Interesse mindestens eines von einer Zungenmitte, einer Zungenwurzel, einem Zungenrand und einer Zungenspitze umfasst.

**8.** Gesundheitsüberwachungsverfahren für eine Person, deren Gesundheitszustand bestimmt werden soll, wobei das Verfahren das Verfahren des Ausziehens einer Region von Interesse aus einem Zungenbild nach einem der Ansprüche 1 bis 7 umfasst, wobei die Datenbank (15) weiter ein von der Person erhaltenes Zungenbild und ein Resultat des Bestimmens eines Gesundheitszustands der Person in Bezug auf mindestens einen charakteristischen Faktor umfasst, und wobei das Zungenbild, das Vorlagen-Zungenbild für die Person und das Resultat des Bestimmens miteinander verknüpft sind, wobei das Verfahren weiter Folgendes umfasst:

Erkennen von mindestens einem charakteristischen Faktor aus der ausgezogenen Region von Interesse; und
Bestimmen des Gesundheitszustands der Person basierend auf einer Änderung eines Zungenzustands, die anhand eines Vergleichs zwischen dem erkannten charakteristischen Faktor und einem aus der Datenbank herausgesuchten charakteristischen Faktor in Bezug auf die Region von Interesse in verschiedenen Gesundheitszuständen erkannt wird.

9. Gesundheitsüberwachungsverfahren nach Anspruch 8, wobei es sich bei dem charakteristischen Faktor um einen Mittelwert von H-, S- oder 1-Werten handelt, die aus einem Histogramm eines HIS-Farbkoordinatensystem für die Region von Interesse erhalten wurden.

10. Computerlesbares Aufzeichnungsmedium, auf dem ein Programm zum Ausführen des Verfahrens nach einem der vorangehenden Ansprüche aufgezeichnet ist.

11. Computerlesbares Aufzeichnungsmedium nach Anspruch 10, wobei jeder Schritt des Verfahrens von einem getrennten Programm ausgeführt wird.

12. Gesundheitsüberwachungsvorrichtung, die ein Zungenbild verwendet, umfassend:

eine Zungenbild-Datenbank (15), in der ein von jeder Person erhaltenes Zungenbild, ein Vorlagen-Zungenbild, in dem Regionen von Interesse für die Person festgelegt sind und ein Resultat des Bestimmens eines Gesundheitszustands der Person in Bezug auf mindestens einen charakteristischen Faktor miteinander verknüpft sind;
eine Zungenbild-Erfassungseinheit (11), die ein Zungenbild von einer Person erfasst, deren Gesundheitszustand bestimmt werden soll;
eine Auszieheinrichtung (23) für eine Region von Interesse, die einen Zungenbereich (402) aus dem von der Zungenbild-Erfassungseinheit (11) bereitgestellten Zungenbild heraustrennt und unter Verwendung eines Vorlagenablgeichs zwischen dem Zungenbereich und einem in der Zungenbild-Datenbank (15) gespeicherten Vorlagen-Zungenbild für die Person eine Region von Interesse aus dem Zungenbereich auszieht;
eine Charakteristik-Auszieheinrichtung (25), die mindestens einen charakteristischen Faktor betreffende Daten in Bezug auf die von der Auszieheinrichtung (23) für eine Region von Interesse ausgezogene Region von Interesse erzeugt;
eine Vergleichseinrichtung (27), welche die den charakteristischen Faktor betreffenden Daten, die in Bezug auf die Region von Interesse von der Charakteristik-Auszieheinrichtung (25) erzeugt wurden, mit einem in der Zungenbild-Datenbank (15) in Bezug auf die Region von Interesse gespeicherten charakteristischen Faktor vergleicht; und
eine Gesundheitszustands-Bestimmungseinrichtung (29), die den Gesundheitszustand der Person basierend auf dem von der Vergleichseinrichtung (27) bereitgestellten Resultat des Vergleichs bestimmt und die Person über das Resultat des Bestimmens informiert.

13. Gesundheitszustands-Überwachungsvorrichtung 12, weiter umfassend eine Farbkorrektureinrichtung (21), die Farbverfälschung des von der Zungenbild-Erfassungseinheit (11) erfassten Zungenbilds unter Verwendung eines Farbprüfers korrigiert und das korrigierte Zungenbild für die Auszieheinrichtung (23) für eine Region von Interesse bereitstellt.

14. Gesundheitsüberwachungsvorrichtung nach Anspruch 12 oder 13, wobei die Auszieheinrichtung (23) für eine Region von Interesse den Vorlagenabgleich unter Verwendung einer Zungenbreite, eines Drehwinkels einer Zunge und einer Zungenlänge, die aus dem Zungenbereich (402) berechnet werden, ausführt.

15. Gesundheitsüberwachungsvorrichtung nach einem der Ansprüche 12 bis 14, wobei die Gesundheitsüberwachungsvorrichtung in eine mobile Kommunikationsausrüstung mit einer Digitalkamera integriert ist.

16. Gesundheitsüberwachungsvorrichtung nach einem der Ansprüche 12 bis 15, wobei die Gesundheitsüberwachungsvorrichtung in einen Personal-Computer mit einer Digitalkamera integriert ist.

17. Gesundheitsüberwachungsvorrichtung nach einem der Ansprüche 12 bis 16, wobei alle der Komponenten, außer der Zungenbild-Erfassungseinheit (11) in einer entfernten Gesundheitspflege-Dienstzentrale implementiert sind und das von der Zungenbild-Erfassungseinheit (11) erfasste Zungenbild durch drahtgebundene oder drahtlose Kommunikation zu der Gesundheitspflege-Dienstzentrale übertragen wird.

18. Gesundheitsüberwachungsvorrichtung nach Anspruch 17, wobei es sich bei der Gesundheitspflege-Dienstzentrale um einen Server eines Unternehmens für mobile Kommunikationsausrüstung oder eines Krankenhauses handelt.

# EP 1 450 287 B1

**Revendications**

1. Procédé d'extraction d'une zone d'intérêt à partir d'une image de la langue d'une personne dont l'état de santé doit être déterminé, le procédé comprenant :

   la construction d'une base de données (15) dans laquelle des images modèles de langue pour une pluralité de personnes sont stockées, y compris une image modèle de langue pour la personne dont l'état de santé doit être déterminé, où chaque image modèle de langue correspond à l'information personnelle et est indiquée avec les zones d'intérêt ;
   la séparation d'une zone de langue (402) à partir d'une image de langue acquise de la personne dont l'état de santé doit être déterminé ;
   la mise en correspondance de la zone de langue séparée (402) avec une image de modèle de langue stockée dans la base de données (15) ; et
   l'extraction de la zone d'intérêt de la zone de langue séparée (402) en utilisant l'image de la langue modèle adaptée.

2. Procédé selon la revendication 1, comprenant en outre la compensation de la distorsion des couleurs de l'image de langue à l'aide d'un correcteur de couleur avant de séparer la zone de langue (402).

3. Procédé selon la revendication 1 ou 2, où la séparation de la zone de langue (402) comprend l'exécution d'un seuillage sur chaque pixel dans l'image de langue en utilisant une valeur de seuil prédéterminée de manière à définir une zone de bouche intérieure et une zone de langue (402) et la séparation de la zone de langue (402) à partir de l'image de langue.

4. Procédé selon la revendication 3, où le seuillage est effectué en utilisant au moins une parmi les valeurs de couleur RVB de chaque pixel dans l'image de langue.

5. Procédé selon la revendication 3, où le seuillage est effectué en utilisant une valeur d'intensité obtenue en convertissant les valeurs de couleur RVB de chaque pixel dans l'image de langue en valeurs de couleur HSI.

6. Procédé selon l'une quelconque des revendications 1 à 5, où la mise en correspondance de la zone de langue séparée (402) comprend :

   le calcul d'une largeur de langue (w), d'un angle de rotation d'une langue (a) et d'une longueur de langue (b) par rapport à la zone de langue séparée (402) ; et
   la mise en correspondance de la zone de langue séparée (402) avec l'image modèle de langue en utilisant la largeur de langue (w), l'angle de rotation (a) et la longueur de la langue (b).

7. Procédé selon l'une quelconque des revendications 1 à 6, où la zone d'intérêt comprend au moins l'un d'un milieu de langue, une racine de langue, un bord de langue et une pointe de langue.

8. Procédé de surveillance de la santé d'une personne dont l'état de santé doit être déterminé, le procédé comprenant le procédé consistant à extraire une zone d'intérêt à partir d'une image de langue selon l'une quelconque des revendications 1 à 7, où la base de données (15) comprend en outre une image de langue obtenue à partir de la personne et un résultat de la détermination d'un état de la personne de la santé par rapport à au moins un élément caractéristique, et où l'image de langue, l'image modèle de langue de la personne et le résultat de la détermination sont reliés les uns aux autres, le procédé comprenant en outre :

   la détection d'au moins un élément caractéristique de la zone extraite d'intérêt; et
   la détermination de l'état de santé de la personne sur la base d'un changement dans un état de langue, qui est détectée à partir d'une comparaison entre le facteur caractéristique détecté et un facteur caractéristique recherché à partir de la base de données par rapport à la zone d'intérêt dans différents états de santé.

9. Procédé de surveillance de santé selon la revendication 8, où le facteur caractéristique est une moyenne des valeurs H, S ou I obtenues à partir d'un histogramme d'un système de coordonnées de couleur HSI de la zone d'intérêt.

10. Support d'enregistrement lisible machine sur lequel est enregistré un programme pour exécuter le procédé selon l'une quelconque des revendications précédentes.

**11.** Support d'enregistrement lisible machine selon la revendication 10, où chaque étape du procédé est effectué par un programme distinct.

**12.** Dispositif de surveillance de la santé en utilisant une image de langue, comprenant :

une base de données d'images de langue (15) dans laquelle une image de langue obtenue à partir de chaque personne, une image modèle de langue dans laquelle des zones d'intérêt sont fixées pour la personne, et un résultat de détermination d'un état de la personne de la santé par rapport à au moins une caractéristique facteur sont reliés les uns aux autres ;
une unité d'acquisition d'image de langue (11) qui acquiert une image de langue d'une personne, dont l'état de santé doit être déterminé ;
un extracteur de zone d'intérêt (23) qui sépare une zone de langue (402) à partir de l'image de langue fournie par l'unité d'acquisition d'image de langue (11) et en extrait une zone d'intérêt à partir de la zone de langue en utilisant un modèle de mise en correspondance entre la zone de langue et une image modèle de langue pour la personne enregistrée dans la base de données d'image de la langue (15) ;
un extracteur de caractéristique (25) qui génère des données concernant au moins un élément caractéristique par rapport à la zone d'intérêt extraite par l'extracteur zone d'intérêt (23) ;
un comparateur (27) qui compare les données relatives à l'élément caractéristique produite par rapport à la zone d'intérêt par l'extracteur de caractéristique (25) avec un facteur caractéristique stocké dans la base de données d'images de langue (15) par rapport à la zone d'intérêt ; et
un élément de détermination de l'état de santé (29) qui détermine l'état de santé de la personne sur la base du résultat de comparaison fourni par le comparateur (27) et informe la personne du résultat de la détermination.

**13.** Appareil de surveillance de santé selon la revendication 12, comprenant en outre un compensateur de couleur (21) qui compense la distorsion des couleurs de l'image de langue acquises par l'unité d'acquisition d'images de langue (11) à l'aide d'un correcteur de couleur et fournit l'image de langue compensée dans l'extracteur de zone d'intérêt (23).

**14.** Appareil de surveillance de santé selon la revendication 12 ou 13, où l'extracteur de zone d'intérêt (23) effectue une mise en correspondance de modèle en utilisant une largeur de langue, un angle de rotation d'une langue et une longueur de langue qui sont calculées à partir de la zone de langue (402).

**15.** Appareil de contrôle de santé selon l'une quelconque des revendications 12 à 14, où l'appareil de surveillance de la santé est intégré à un équipement de communication mobile avec un appareil photo numérique.

**16.** Appareil de contrôle de santé selon l'une quelconque des revendications 12 à 15, où l'appareil de surveillance de la santé est intégré à un ordinateur personnel avec un appareil photo numérique.

**17.** Appareil de contrôle de santé selon l'une quelconque des revendications 12 à 16, où tous les composants à l'exception de l'unité d'acquisition d'images de langue (11) sont mis en oeuvre dans un centre de services de soins de santé à distance et l'image de la langue acquise par l'unité d'acquisition d'images de langue (11) est transmise au centre de services de soins de santé par un communication filaire ou sans fil.

**18.** Appareil de surveillance de la santé selon la revendication 17, où le centre de services de soins de santé est un serveur d'une société de matériel de communication mobile ou un hôpital.

# FIG. 1

```
┌─────────────────────────┐
│     TONGUE IMAGE        │
│   ACQUISITION UNIT      │──11
└─────────────────────────┘
            │
            ▼
┌─────────────────────────┐
│     TONGUE IMAGE        │
│   PROCESSING UNIT       │──13
└─────────────────────────┘
            ↕
┌─────────────────────────┐
│     TONGUE IMAGE        │
│       DATABASE          │──15
└─────────────────────────┘
```

# FIG. 2

13

| 21 | 23 | 25 | 27 | 29 |
|---|---|---|---|---|

TONGUE
IMAGE
→ COLOR
COMPENSATOR
→ REGION-OF
-INTEREST
EXTRACTOR
→ CHARACTERISTIC
EXTRACTOR
→ COMPARATOR
→ HEALTH
STATE
DETERMINER

TONGUE
IMAGE
DATABASE —15

# FIG. 3

```
        ( START )
            |
            v
+------------------------+
|    GENERATION OF       |--- 31
|   TEMPLATE IMAGE       |
+------------------------+
            |
            v
+------------------------+
|    THRESHOLDING        |---33
+------------------------+
            |
            v
+------------------------+
|    MATCHING OF         |---35
|   TEMPLATE IMAGE       |
+------------------------+
            |
            v
+------------------------+
|    EXTRACTION OF       |---37
|  REGION OF INTEREST    |
+------------------------+
            |
            v
        ( END )
```

# FIG. 4A

# FIG. 4B

## FIG. 4C

## FIG. 4D

# FIG. 5

# FIG. 6A

# FIG. 6B

# FIG. 7A

71

# FIG. 7B

73

# FIG. 8A

31

# FIG. 8B

83

# FIG. 9A

# FIG. 9B

## FIG. 10A

## FIG. 10B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001314376 A

**Non-patent literature cited in the description**

- **CHUANG-CINE CHIU.** A Novel Approach Based on Computerized image Analysis for Traditional Chinese Medical Diagnosis of the Tongue. *Computer Methods and Programs in Biomedicine,* 2000, vol. 61, 77-89
- **YANG CAI.** A novel Imaging System for Tongue Inspection. *IEEE Instrumentation and Measurement Technology Conference,* 2002, 159-163
- **YAO ; PAOTIA.** Comparison of TCM Tongue Images with Gastroscopy Images. Shangdong S&T Publisher, 1996
- **TADASHI ; WATSUJI et al.** Medical Application of Fuzzy Theory to the Diagnostic System of Tongue Inspection in Traditional Chinese Medicine. *IEEE International Fuzzy Systems Conference Proceedings,* 1999, 145-148
- **C.H. LI ; P.C. YUEN.** Tongue image matching using color content. *Pattern Recognition,* 2002, vol. 35, 407-419